# EUROPEAN PATENT SPECIFICATION

(11) **EP 2 542 096 B1**
(45) Date of publication and mention of the grant of the patent: **15.07.2015**
(21) Application number: 11708188.5
(22) Date of filing: 28.02.2011
(51) Int. Cl.: A23G 1/42, A23G 1/54, A61K 9/00, A61P 39/06, A61P 27/02

(54) **NUTRACEUTICAL CHOCOLATE OR COMPOUND CHOCOLATE PRODUCT**
NÄHRERGÄNZUNGSMITTEL AUF BASIS VON SCHOKOLADE ODER DERIVAT DAVON
COMPLEMENT ALIMENTAIRE SOUS FORME DE CHOCOLAT OU PRODUIT DERIVE

(30) Priority: 05.03.2010 US 282594 P
(43) Date of publication of application: 09.01.2013
(73) Proprietor: Ophthalmopharma AG, 6060 Samen (CH)
(72) Inventor: GERON, Mordechai, CH-1009 PuII (CH); DE CHADAREVIAN, Samir, 8044 Zürich (CH)
(74) Representative: Teuten, Andrew John
(86) International application number: PCT/EP2011/000999
(87) International publication number: WO 2011/107259

(56) References cited:
- WO-A1-2010/125516
- WO-A1-2011/022318
- WO-A2-2006/063219
- WO-A2-2009/037562
- US-A- 4 749 575
- US-A1- 2009 155 381
- US-B1- 6 582 721
- US-B2- 6 660 297

## Description

### Field of the Invention

The present invention relates to novel nutraceutical chocolate or compound chocolate formulations comprising added functional ingredients in amounts believed to be effective in reducing the severity or slowing development of diseases of the eye such as macular degeneration and preventing other age-related deficiencies.

### Background of the Invention

The retina is the layer of nerve cells at the back of the eye, which nerve cells convert light into signals that are sent to the brain. In humans and other primates (but not in most other mammals), the macula is a small yellowish circular area in the retina, positioned at the center of the field of vision. It provides fine-resolution vision in the center of the visual field, and it is essential to good vision. Patients who suffer from macular degeneration often lose the ability to read, recognize faces, drive, or walk safely on unfamiliar routes. Macular degeneration is the leading cause of severe vision loss and functional blindness among the elderly, and its rates are increasing as the population ages and dietary patterns shift away from dark green vegetables toward more fatty foods. The disease, including approaches for preventing or treating it, is described in many books and articles. D'Amato R et al, Macular Degeneration: The Latest Scientific Discoveries and Treatments for Preserving Your Sight (Walker & Company, 2000); Lim JI, editor, Age-Related Macular Degeneration (Marcel Dekker, 2002); Byrne S et al, "Current concepts and recent advances in the management of age-related macular degeneration," Ir J Med Sci 2003 October-December; 172(4): 185-90; Blodi BA, "Nutritional supplements in the prevention of age-related macular degeneration," Insight 2004 January-March; 29(1): 15-6; Zarbin MA, "Current concepts in the pathogenesis of age-related macular degeneration," Arch Ophthalmol 2004 April; 122(4): 598-614.

While medications for treating macular degeneration have been developed (e.g., Lucentis™, approved in 2006 by the U.S. FDA for vascular age-related macular degeneration) and continue to be developed, interest has remained high in the use of nutritional supplements for preventing or reducing progression of the disease. This interest dates back to the 1990s when the U.S. National Eye Institute organized and carried out a major clinical trial involving thousands of patients who were suffering from or were at high risk of developing macular degeneration and/or cataracts. Macular degeneration and cataracts are clearly related to aging. They strongly increase in frequency and severity as people pass beyond the age of about 60, and they rarely if ever occur in anyone under the age of about 50, except in people who have specific genetic defects affecting their eyes. Accordingly, the trial was called the "Age-Related Eye Disease Study", abbreviated as AREDS. As a follow-up study called AREDS-2 has been initiated, the study carried out in the 1990's is referred to herein as AREDS-1. In the AREDS-1 study, participants were divided into four treatment arms, which were anti-oxidants alone, zinc alone, anti-oxidants plus zinc, or nothing (controls). In each treatment arm, participants were divided into four categories, depending on their eye health when they entered the study. Category 1, at the low end of the scale, contained people with no apparent signs of macular problems. Category 4, at the high end of the scale, contained people with serious problems in one eye while the other eye remained sufficiently free of advanced problems which permitted monitoring and measuring of subsequent declines after the person began taking supplements. Daily anti-oxidants administered were 500 mg of vitamin C, 295 mg of vitamin E and 15 mg of beta-carotene. Daily "zinc" was given as 80 mg of zinc in the form of zinc oxide in combination with 2 mg of copper in the form of cupric oxide for preventing anemia.

The main results of AREDS-1 were published in two articles in the October 2001 issue of Archives of Ophthalmology. Macular degeneration data were reported in AREDS Report Number 8 (Arch Ophthalmol 119: 1417-1436), whereas cataract data were reported in AREDS Report Number 9 (Arch Ophthalmol 119: 1439-1452). While AREDS Report 8 should be consulted for details, its results generally can be summarized as follows: (1) Test patients who suffered from moderate or advanced macular degeneration, and who received Vitamins A, C, and E but no zinc, showed some positive results, but the indicators did not rise to a level of statistical significance. (2) Test patients who suffered from moderate or advanced macular degeneration, and who received zinc but no antioxidant vitamins, also showed some positive results, but the indicators did not reach statistical significance. (3) A third set of test patients who suffered from moderate or advanced macular degeneration, and who received both zinc and antioxidants, showed positive results that reached a level of statistical significance. U.S. Pat. No. 6,660,297 by Bartels et al., which patent is assigned to Bausch & Lomb, claims certain nutritional supplements that are based on the compositions tested in AREDS-1. A product being sold by Bausch & Lomb under that patent is called OCUVITE PRESERVISION^{™}. A closely similar formulation called ICAPS AREDS^{™} is sold by Alcon Laboratories.

The current formulations are largely offered in the form of pills or gel caps, i.e., in formats typical for medications. Hence, these nutritional supplement formulations suffer from the same problem as typical medications, i.e., unsatisfactory patient compliance. To alleviate this problem, the present applicants have developed chocolate formulations comprising functional ingredients vitamin C, vitamin E, lutein, zeaxanthin, zinc, copper and, optionally, a xanthophyll, preferably a combination of lutein and zeaxanthin, chocolate being the arguably most craved for food. In the course of this effort, problems needed to be resolved that related to chemical instability of functional ingredients caused by their introduction into chocolate or occurring on account of the simultaneous presence of two or more such ingredients in chocolate. Further problems related to unpleasant taste or mouth feel generated by addition of certain ingredients to chocolate. In addition, it was desirable that the functional ingredients be present in chocolate formulations in high concentration so as to minimize the caloric impact of daily consumption of the nutraceutical product. A first solution to the problems was provided in WO 2009/037562. In the approach described in this publication, vitamin C, zinc and copper are introduced into a food, including a chocolate, such that the vitamin C is present as a fatty acid or ethyl ester and the metals as fatty acids salts. While the latter approach represents a significant advance by providing nutraceutical foods with improved oxidative stability, its realization is encumbered by the fact that fatty acid salts of copper and zinc are not currently approved food additives. The present invention relates to new chocolate formulations comprising the afore-mentioned functional ingredients in suitably concentrated form, in which formulations the functional ingredients are chemically stable and do not cause a taste or mouthfeel detectable by a typical consumer.

### Summary of the Invention

The present invention relates to a chocolate or compound chocolate product comprising added functional ingredients including vitamin C, vitamin E, zinc, copper and, optionally, xanthophyll. The chocolate or chocolate compound product has a casing and a filling wherein at least the vitamin C, the vitamin E and the xanthophyll, if added, are comprised in the filling. One or both of the zinc and the copper are present in the casing. The ingredients contained in the chocolate or compound chocolate product of the invention are essentially chemically stable and do not produce a textural or taste effect detectable by a typical consumer during the shelf life of the product, with the proviso that the concentration of added vitamin C does not exceed about 75 mg per gram of product, that of added vitamin E about 45 mg per gram of product, that of added zinc about 3 mg per gram of product, that of added copper about 0.4 mg per gram of product and that of added xanthophyll about 2.6 mg per gram of chocolate of compound chocolate product. The latter quantities relate to the active forms of the ingredients. A typical consumer is a consumer with average sensitivities. For example, if 4 of 5 consumers do not detect any textural or taste effect in the product of the invention, each of these 4 consumers will be said to be a typical consumer.

In one embodiment, the chocolate or compound chocolate product of the invention has a shelf life of at least three months.

In another embodiment, the chocolate or compound chocolate product further comprises one or more added flavors, whereby the flavors are included in the filling. The flavors added include but are not limited to mint, orange, spicy orange, caramel, butter scotch, vanilla, coffee, cinnamon, pepper, chili, raspberry, strawberry, ginger, hazelnut, honey, lemon, lime, papaya, pistachio, pecan, tamarind, tangerine or walnut, whereby the flavors can be added in any form, including in the form of essences, extracts or in dry form.

In a specific embodiment of the chocolate or compound chocolate product of the invention the vitamin C is added as a palmityl, lauryl, cocoyl, oleyl or stearyl ester. More preferably, the vitamin C included is ascorbyl palmitate. If a xanthophyll is added to the chocolate or compound chocolate product of the invention, this xanthophyll preferably is either lutein or a combination of lutein and zeaxanthin.

In a preferred embodiment, the vitamin E, the copper, the xanthophyll (if present) and, optionally, the zinc are added to the chocolate or compound chocolate product as encapsulated ingredients. Fluid bed coating processes are the preferred methods for encapsulating ingredients included in the chocolate or compound chocolate product of the invention. In a more particular embodiment, the mean particle size of the added encapsulated functional ingredients is greater than about 100 µm. The vitamin E and the xanthophyll to be added to the chocolate or compound chocolate product can be encapsulated separately or can be co-encapsulated, i.e., encapsulated together in a single process. Similarly, if zinc is included as an encapsulated ingredient, the copper and zinc can be co-encapsulated.

In the chocolate or compound chocolate product of the invention one or both of the zinc and the copper are present in the casing.

In the chocolate or compound chocolate product of the invention zinc is added as an encapsulated or non-encapsulated zinc oxide or an encapsulated or non-encapsulated zinc salt in the form of a sulfate, chloride, acetate, gluconate, ascorbate, citrate, aspartate, picolinate, orotate or transferrin salt. Copper is added preferably in the form of encapsulated cupric oxide, or encapsulated cupric L-alaninate, cupric carbonate, cupric chloride, cupric citrate, cupric gluconate, cupric glycinate, cupric salicylate, cupric sulfate or cupric tartrate. Vitamin E is added preferably in the form of encapsulated DL-α-tocopheryl acetate, DL-α-tocopherol, d-α-tocopheryl acetate, d-α-tocopherol or vitamin E isolated from natural sources.

In particular embodiments, the casing as well as the filling of the chocolate or compound chocolate product of the invention can be either dark chocolate, milk chocolate, white chocolate or compound chocolate of dark, milk, or white chocolate flavor. Preferably, the casing is dark or milk chocolate, and the filling is compound chocolate of milk chocolate flavor.

In specific low glycemic index formulations of the chocolate or compound chocolate product of the invention, the sugar normally present in chocolate-based products, i.e., sucrose and, in milk-based products, lactose, is supplemented or substituted in whole or in part by one or more of an alternative carbohydrate, a bulk sweetener and an intense sweetener. Alternative carbohydrates that can be used in such low glycemic index formulations include fructose, tagatose, lactose, isomaltose, trehalose, polydextrose and inulin. Bulk sweeteners that can be employed include sorbitol, mannitol, xylitol, erythritol, maltitol, isomalt and lactitol, and useful intense sweeteners comprise Acesulfamine potassium, Alitame, Aspartame, Aspartate-acesulfame salt, Cyclamate, Neotame, Saccharin, Stevia, Sucralose and Thaumatin.

Particular embodiments relate to daily dosage forms of the chocolate or compound chocolate product of the invention, whereby a single chocolate or compound chocolate product or a plurality of chocolates or compound chocolate products comprise 12-2000 mg of added vitamin C, 2.5-1000 mg added vitamin E, 1.5-40 mg added zinc, 0.15-10 mg added copper and, optionally, 1.5-33 mg added xanthophyll(s), whereby the amounts claimed refer to the active forms of the ingredients. Preferably such daily dosage forms comprise 180-1000 mg of added vitamin C, 30-500 mg added vitamin E, 5-30 mg added zinc, 1-2 mg added copper and 7-20 mg added xanthophylls. A more preferable daily dosage form provides for 500 mg of added vitamin C, 268 mg added vitamin E, 25 mg added zinc, 1.6 mg added copper and 12 mg added xanthophylls.

Another embodiment relates to a method of producing a chocolate or compound chocolate product of the invention comprising providing a chocolate or compound chocolate product and the further step of adding appropriate amounts of functional ingredients vitamin C, vitamin E, zinc, copper and, optionally, xanthophyll to liquid chocolate or compound chocolate masses used for preparing casing and filling, whereby the additional step occurs subsequent to all milling steps. Optionally, said step of providing a chocolate or compound chocolate product comprising making a chocolate or compound chocolate product; optionally the steps of providing or making a chocolate or compound chocolate product comprise all steps normally used for making a chocolate or compound chocolate product.

The invention also relates to a method for dietetic prevention and/or treatment of diseases of the eye, in particular age-related macular degeneration or cataract formation, or for generally supporting eye health, comprising administering daily to a subject in need of such prevention or treatment a daily dose of chocolate or compound chocolate product, whereby such daily dose may be present in a single chocolate or compound chocolate product or in a plurality of chocolates or compound chocolate products. The method can also include co-administration of another nutritional supplement. Preferably co-administered is a nutritional supplement providing omega-3 fatty acids in amounts deemed appropriate for daily consumption. Also considered to be within the scope of the invention is a method for dietetic prevention and/or treatment of diseases of the eye, in particular age-related macular degeneration or cataract formation, or for supporting general eye health, comprising daily administration to a subject in need of such prevention or treatment of a daily dose of chocolate or compound chocolate product of the invention or another nutraceutical supplement capable of delivering one or more of the same functional ingredients as the chocolate or compound chocolate product, whereby the subject is administered the single chocolate or compound chocolate product or the plurality of chocolate or compound chocolate products at least once a week. In the latter methods, if used therapeutically, chocolate or compound chocolate product of the invention is used as a medicament for the treatment or prevention of diseases of the eye such as macular degeneration or cataract formation.

A final embodiment relates to kits for weekly, biweekly or monthly use, which kits comprise numbers of daily doses of chocolates or chocolate compound products of the invention commensurate with the intended therapy interval.

### Detailed Description of the Invention

The present invention relates to new dosage forms of zinc and antioxidants believed to be effective in retardation of progression or prevention of macular degeneration and other age-related deficiencies in elderly people. This belief is based on the results of the AREDS-1 study discussed before. Supplement formulations comprising zinc and antioxidants on the market are pills or gel caps, which are formulations that suffer from the same problem of unsatisfactory patient compliance as do many prescription and non-prescription drugs. It is presumed that regular consumption is a precondition for maximizing the medical benefits of the supplement. The applicant proposes that patients will be more likely to comply if zinc and antioxidants are provided in a food that is both highly attractive or even craved for and is devoid of any specific and unpleasant taste associated with added functional ingredients, e.g., the acidic taste vitamin C, the burning and bitter tastes of heavy metal ions, the unpleasant taste of oxidation or degradation products of xanthophylls or vitamin E, of unappealing texture due to uneven distribution of components, e.g., pockets of insoluble metal oxides, etc., and of grittiness and unpleasant mouthfeel due to addition of excess ingredients in the form of large particles. Therefore, the present invention relates to a nutraceutical chocolate or compound chocolate comprising vitamin C, vitamin E, zinc, copper, and, preferably, also one or more xanthophylls in a format in which these functional ingredients are stable and do not produce any detectable taste or mouthfeel. Applicant believes that patient compliance can be improved, if patients are offered a nutraceutical chocolate or compound chocolate product that has the taste of high-quality chocolate. Chocolates containing vitamins and minerals have been introduced in the market before (e.g., Nestrovit by Nestlé Suisse SA, Egmovit by Iromedica SA), but these products do not satisfy the above criteria of being perceived as high-quality chocolate products devoid of any taste ascribable to the functional ingredients contained in them.

The mixture of functional ingredients that was administered daily in the AREDS-1 study was comprised of 500 mg of vitamin C, 295 mg of vitamin E, 15 mg of beta-carotene, 80 mg of zinc in the form of zinc oxide and 2 mg of copper in the form of cupric oxide. AREDS Report Number 8. Zinc and the anti-oxidants are believed to be the active elements of the formula that protect against progression of macular degeneration. Copper is an essential trace metal. Copper deficiency results in anemia, cardiac abnormalities such as blood vessel and heart rupture, abnormal EKG's, and elevated levels of serum cholesterol, triglycerides and glucose. A lifetime of marginal dietary copper in humans is thought to lead to heart disease. Overt symptoms in adults are rare, but may occur with long-term shortage or, in those who regularly consume zinc supplements. Zinc is known to reduce copper status. Vitamin C supplementation also results in decreased copper status. In rates, large doses of vitamin C can lead to copper deficiency. Therefore, copper was included in the AREDS formula that comprised elevated concentrations of both zinc and vitamin C for preventing copper deficiency.

Very high dosages of vitamins C and E may not be safe. For example, studies have indicated that post-menopausal women who suffer from coronary artery disease should not take high dosages of vitamins C or E. In recent years, it has been realized that beta-carotene is not deposited in the macula in human eyes. Therefore, a consensus has emerged among eye and vision researchers that high dosages of beta-carotene do not offer any realistic promise of providing any substantial benefit or protection against macular degeneration. Moreover, beta-carotene actually reverses its anti-oxidant activity and becomes a pro-oxidant, when unusually high concentrations of oxygen are present (e.g., Burton, GW et al 1984 "Beta-carotene: An unusual type of lipid antioxidant," Science 224: 569-573). Such oxygen concentrations are not present in most tissues and fluids in the body; however, since the lungs interact directly with oxygen in the air that is breathed, beta-carotene may act as a damaging pro-oxidant, rather than a beneficial anti-oxidant, in lung tissues. As an apparent result of its damaging pro-oxidant activity at high oxygen concentrations, large clinical trials have convincingly shown that high-dosage beta-carotene, instead of being useful and protective, actually increases the risks of lung cancer among smokers. This clearly and unmistakably occurs among smokers, and it may also be happening to a lesser extent among non-smokers. Accordingly, the Bartels et al '297' patent specifically stated that beta-carotene could be deleted from the anti-oxidant combination that was tested in the AREDS-1 trial, and could be replaced by either one or both of two other carotenoids, lutein and zeaxanthin, which xanthophylls are actually present in the retina. Zinc ions were administered as zinc oxide in AREDS-1. Dosages used in the trial are believed to be 80 mg/day of zinc oxide; however, the published AREDS reports referred to "zinc, 80 mg, as zinc oxide". This raises the question as to whether the "80 mg" dosage referred to elemental zinc (molecular weight 65.4), or zinc oxide (molecular weight 81.4). Current "Recommended Daily Allowance" values (abbreviated RDA; published in 2001 by the U.S. Food and Nutrition Board (part of the Institute of Medicine)) for elemental zinc are 8 milligrams per day for adult females and 11 milligrams per day for adult males. Therefore, a daily dosage of 69.6 mg of elemental zinc, from OCUVITE PRESERVISION pills alone, without considering other dietary sources, is actually 870% of the RDA for women, and 633% of the RDA for men. In addition to RDA numbers, the Institute of Medicine has recently adopted and issued "Tolerable Upper Intake Levels" (abbreviated "UL"s) for various nutrients. For zinc, the UL was set in 2001 at 40 mg/day for both men and women. Therefore, the amount of zinc in OCUVITE PRESERVISION, by itself, appears to be nearly twice as high as the UL set by the Institute of Medicine, and the surplus becomes even higher if additional zinc intake in the diet is also taken into account. As discussed in U.S. Patent Publication No. 20060039954 by Gierhart et al, there are numerous publications suggesting that extra-heavy dosages of zinc may cause serious risks, not among all elderly consumers, but among sufficient numbers to create major concerns. Three specific concerns involve neurology. The first focuses on Alzheimer's disease and beta-amyloid plaques. The second concern relates to the severity of brain damage and permanent impairment following a stroke, cardiac arrest, or other crisis that assaults the brain. The third concern focuses on cognitive impairments seen in animals that were fed heavy dosages of zinc. Heavy zinc intake may also trigger or stimulate the growth of prostate cancer among middle-aged and elderly men. In addition, zinc has been discovered in high concentrations in unwanted deposits in human retinas called "drusen". This observation suggests the possibility that heavy zinc intake may accelerate the formation and growth of those unwanted deposits, which can disrupt the retina and damage vision. Apparently, the above risks were not recognized or considered by the people who organized and conducted the AREDS-1 trial or by the companies that are selling formulations with heavy dosages of zinc to elderly consumers for the prevention or treatment of macular degeneration. It has been argued that future formulations to be offered to elderly customers for the prevention or treatment of macular degeneration should have levels of zinc, vitamin C and vitamin E that do not exceed the UL values, and should include the xanthophylls lutein and zeaxanthin instead of beta-carotene. If the intake through food sources is considered, levels of zinc, vitamin C and vitamin E ideally should not exceed the ULS values (tolerable upper intake levels from supplements). As is argued in the '954' patent publication, applying principles of homeostasis and diminishing marginal utility, such reduction of dosages of zinc, and vitamins C and E should not diminish markedly the ocular benefits of the formulations, and substitution of xanthophylls, i.e., lutein or zeaxanthin, for beta-carotene should enhance their effectiveness.

The present invention relates to a chocolate or a compound chocolate product, or a plurality of chocolate or compound chocolate products, that comprises vitamin C, vitamin E, zinc, copper and, optionally, a xanthophyll (preferably lutein or a combination of lutein and zeaxanthin). A daily dose of chocolate(s) or compound chocolate product(s) comprises 12-2000 mg of added vitamin C, 2.5-1000 mg added vitamin E (tocopherol equivalents = TE), 1.5-40 mg added zinc, 0.15-10 mg added copper, and 1.5-33 mg added xanthophyll. More preferably, a daily dose comprises 180-1000 mg of added vitamin C, 30-500 mg added vitamin E, 5-30 mg added zinc, 1-2 mg added copper and 7-20 mg added xanthophylls. In a particularly preferred specific embodiment, a daily dose of chocolate(s) or compound chocolate product(s) comprises 500 mg of added vitamin C, 294 mg of vitamin E, 25 mg of zinc, 1.6 mg of copper and 12 mg of xanthophylls (preferably a combination of lutein and of zeaxanthin). The amounts given above relate to the "elemental" or "active" forms of the ingredients. A daily dose of the latter formulation is said to comprise 500 mg of vitamin C, i.e., an amount corresponding to 500 mg of ascorbic acid. As also discussed below, vitamin C is preferably added as an ascorbyl ester. In order to arrive at an elemental dose of 500 mg vitamin C, 1175.5 mg of an ascorbyl palmitate would have to be included. Analogously, if zinc were introduced as a zinc gluconate, 174.25 mg would have to be added to provide an elemental dose of 25 mg zinc. It is noted that it should be possible in most countries to offer formulations containing ingredient levels that are within the ranges of functional ingredients given above. However, it cannot be excluded that certain countries or organizations may require lower doses of one or more ingredients.

Chocolate is a complex product of cacao beans and has been prepared in various forms and consumed for hundreds of years. Methods of preparation and properties of chocolate products are described in a number of publications. See, e.g., Beckett ST 2008 "The Science of Chocolate", The Royal Society of Chemistry, Thomas Graham House, Science Park, Milton Road, Cambridge CB4 OWF, UK; "Industrial Chocolate Manufacture and Use" (Beckett ST, ed.) Fourth edition 2009 John Wiley & Sons Ltd., Chichester, West Sussex PO19 8SQ, UK. To prepare chocolate, cacao beans harvested from pods of tropical cocoa trees (Theobroma cacao L.) are fermented for several days. Typical fermentation processes occur on the outside of the bean. During the fermentation processes, the temperature in the interior of the beans rises dramatically, resulting in the killing of the bean and the decomposition of the bean's food reserves to sugar and acids, the latter being the precursors of the typical chocolate flavor. The beans are subsequently dried to reduce moisture in the bean to about 7-8%, which is below the level that sustains growth of moulds. The roasting process serves to develop the typical chocolate flavor as well as to kill microbial contaminants such as salmonellae. While traditionally whole beans were roasted, alternatives were developed to overcome the difficulty of achieving even roasting of whole beans of different sizes. One such process uses cocoa nibs after winnowing, i.e., separation of the shell and some of the germ from the rest of the bean. Alternatively, the nib is milled to produce cocoa mass or cocoa liquor, if melted, which is then subjected to roasting. In the presence of heat, moisture and reducing sugar (glucose) the Maillard reaction occurs which is responsible for much of the flavor production that takes place during roasting. Cocoa butter is produced from cocoa liquor by pressing, using specially adapted presses. The remainder, the cocoa press cake can be milled to produce cocoa powder. Milling of cocoa nibs and sugar results in a hard crumbly material. Dark eating chocolate is produced by the addition of cocoa butter to the latter two ingredients. When preparing eating chocolate, the nonfat components, here sugar, and the fat components can be milled separately or combined. The fat components or, more conventionally, the combined chocolate mixture is milled as a liquid mass at elevated temperature. Milk chocolate also contains a milk component, typically added in the form of skim milk or full cream milk powder. White chocolate is traditionally made from sugar, milk powder and cocoa butter. The milling process is critical to reduce the size of particles contained in the chocolate product to below about 30 µm as well as to ensure that the particles are coated with fat. If particle size is larger than about 30 µm, the product has a gritty texture or mouthfeel. Subsequent to extensive milling to reduce particle size, the chocolate mass is subjected to the so-called conching process. While this process initially served to further reduce particle size, milling is currently sufficiently efficient so that conching hardly causes any further particle breakage. The process currently is used primarily for flavor development and reduction of viscosity of the liquid chocolate. Chocolate that leaves the conching process at a temperature of at least about 40°C is subjected to tempering, which is a process that produces a sufficient amount of seed fat crystals that have the desired crystal form. To achieve this, chocolate is cooled to induce crystal formation. The temperature is then raised to about 30°C to melt undesired crystals (mainly form IV), favoring the desired crystal form (form V). Thereafter, the still liquid chocolate is used for manufacturing the final chocolate product. The chocolate may be poured into a mould to produce a chocolate bar upon setting of the chocolate. Shell moulding may be employed to produce products that have a chocolate shell surrounding a solid or semisolid center. In the case of Easter eggs, the center may be left hollow. The enrobing process involves a miniature chocolate waterfall that covers centers as they pass through the waterfall. Mars Bar^{R}, After Eight^{R} and Cadbury's Crunchie^{R} are made this way. Other products are manufactured using a panning process. These include chocolate-coated products containing a nut or a dried fruit, or sugar-coated products containing a chocolate center such as Smarties^{R} and M&Ms^{R}.

Chemically, chocolates are complex products. A recipe for a high-quality milk chocolate lists the following components:

**Table 1: High-quality milk chocolate bar (adapted from "Industrial Chocolate Manufacture and Use" (Beckett ST, ed.) Fourth edition (2009))**

| Component | % |
|---|---|
| Sugar | 42 |
| Full cream milk powder | 25 |
| Cocoa butter | 24.5 |
| Cocoa mass (cocoa liquor) | 8 |
| Lecithin | 0.5 |
| Approximate total fat (milk + cocoa butter) | 35 |

With the possible exception of sugar (if only sucrose is used), all components listed are themselves complex mixtures of different compounds. Commercial lecithin as used in the food industry is a mixture of phospholipids, i.e., phosphatidyl choline, phosphatidyl inositol, phosphatidyl ethanolamine and phosphatidic acid. High fat milk powder includes 15.6% proteins (caseins and whey proteins), 54.8% fat, 24.3% lactose, 3.5% minerals and 1.8% moisture.

Milk fat is itself a mixture of different lipids and some other fat-soluble compounds, including flavor compounds. See Table 2 below.

**Table 2: Components of milk fat (adapted from "Industrial Chocolate Manufacture and Use" (Beckett ST, ed.) Fourth edition (2009))**

| Component | Weight % |
|---|---|
| Triglycerides | 98.3 |
| Diglycerides | 0.3 |
| Monoglycerides | 0.1 |
| Free fatty acids | 0.1 |
| Phospholipids | 0.8 |
| Sterol | 0.35 |
| Carotenoid | Trace |
| Vitamins (mainly A, D and E) | Trace |
| Flavor compounds | Trace |

The fatty acid composition of milk fat is shown in Table 3.

**Table 3: Fatty acid composition of milk fat (adapted from "Industrial Chocolate Manufacture and Use" (Beckett, ST, ed.) Fourth edition (2009))**

| | |
|---|---|
| C4:0 Butyric | 4.1 |
| C6:0 Caproic | 2.4 |
| C8:0 Caprylic | 1.4 |
| C10:0 Capric | 2.9 |
| C10:1 Caproleic | 0.3 |
| C12:0 Lauric | 3.5 |
| C14 :0 Myristic | 11.4 |
| C16 :0 Palmitic | 23.2 |
| C18:0 Stearic | 12.4 |
| C18:1 Oleic | 25.2 |
| C18:2 Linoleic | 2.6 |
| C18:3 Linolenic | 0.9 |
| Others | 10.0 |

Cocoa liquor is made by the milling of cocoa nibs. Cocoa nibs have the following composition:

**Table 4: Composition of cocoa nibs (adapted from "Industrial Chocolate Manufacture and Use" (Beckett ST, ed.) Fourth edition (2009))**

| Component | Mean % | Range % |
|---|---|---|
| Water/moisture | 3.7 | 2-5 |
| Fat (cocoa butter and shell fat) | 53.5 | 48-57 |
| Protein | 12.7 | 11-16 |
| Starch | 6.7 | 6-9 |
| Fiber | 2.5 | 2.1-3.2 |
| Ash | 2.9 | 2.6-4.2 |
| Theobromine | 1.3 | 0.8-1.4 |
| Caffeine | 0.22 | 0.1-0.7 |

Cocoa butter is pressed from cocoa liquor. Cocoa butter is essentially a mixture of triglycerides as detailed in Table 5. The fatty acid spectrum is shown in Table 6.

**Table 5: Trigycerides in a West African cocoa butter (adapted from "Industrial Chocolate Manufacture and Use" (Beckett ST, ed.) Fourth edition (2009))**

| Triglyceride type | Fraction (%) |
|---|---|
| SSS | 1.0 |
| SOS | 76.8 |
| SSO | 0.4 |
| SLiS | 6.9 |
| SOO | 8.4 |
| OOO | 6.1 |

| | |
|---|---|
| S: saturated fatty acid (mainly palmitic and stearic); O: oleic; Li: linolenic | |

Furthermore, chocolates also contain non-negligible amounts of minerals and vitamins as detailed in Table 7 below. An important other large group of chemicals that originate from cocoa are polyphenols. A 100 g milk chocolate bar may contain as much as 750 mg of these compounds. Cocoa is particularly rich in flavonols, a subclass of a group of polyphenols known as flavonoids. Cocoa contains monomeric flavonols epicatechin and catechin and larger oligomeric flavonols, the procyanidins. These compounds are chemically reactive and act as anti-oxidants.

**Table 6: Fatty acid composition of a West African cocoa butter (adapted from "Industrial Chocolate Manufacture and Use" (Beckett ST, ed.) Fourth edition (2009))**

| Fatty Acid | Formula | Fraction (%) |
|---|---|---|
| Myristic | C14:0 | 0.1 |
| Palmitic | C16:0 | 26.0 |
| Palmitoleic | C16:1 | 0.3 |
| Stearic | C18:0 | 34.4 |
| Oleic | C18:1 | 34.8 |
| Linoleic | C18:2 | 3.0 |
| Linolenic | C18:3 | 0.2 |
| Arachidic | C20:0 | 1.0 |
| Behenic | C22:0 | 0.2 |

Finally, chocolates also contain large numbers of flavor compounds resulting from fermentation or roasting of cocoa or from subsequent chocolate processing (e.g., conching), which compounds typically are present in minor amounts. Specific flavoring compounds may also be added to chocolate. In all, chocolates are said to contain around 800 distinct chemical compounds. Compounds identified chemically are described summarily in Table 8.

**Table 7 Mineral and vitamin content of different chocolates (per 100 g) (adapted from "Industrial Chocolate Manufacture and Use" (Beckett ST, ed.) Fourth edition (2009))**

| Compound | Dark chocolate | Milk chocolate | White chocolate |
|---|---|---|---|
| Iron, mg | 2.3 | 1.4 | 0.2 |
| Copper, mg | 0.71 | 0.24 | Trace |
| Zinc, mg | 2.3 | 1.1 | 0.9 |
| Manganese, mg | 0.63 | 0.22 | 0.02 |
| Calcium, mg | 33 | 220 | 270 |
| Magnesium, mg | 89 | 50 | 26 |
| Phosphorus, mg | 140 | 220 | 230 |
| Potassium, mg | 300 | 390 | 350 |
| Thiamin, mg | 0.04 | 0.07 | 0.08 |
| Riboflavin, mg | 0.06 | 0.49 | 0.49 |
| Niacin, mg | 0.4 | 0.4 | 0.2 |
| Vitamin B12, µg | 0 | 1.0 | 1.0 |
| Vitamin E, mg | 1.44 | 0.45 | 1.14 |

**Table 8: Chemical compounds identified in chocolate**

| Compounds | Aliphatic type | Aromatic type | Heterocycles | |
|---|---|---|---|---|
| Hydrocarbons | 15 | 32 | Pyrroles | 10 |
| Alcohols | 23 | 5 | Pyridines | 8 |
| Aldehydes | 18 | 6 | Quinoline | 1 |
| Ketones | 25 | 5 | Pyrazines | 74 |
| Esters | 44 | 12 | Quinoxalines | 3 |
| Ethers | 8 | 3 | Oxazoles | 4 |
| Nitrogen cps. | 9 | 4 | Furans | 19 |
| Sulfur cps. | 12 | 2 | Pyrones | 4 |
| Acids | 22 | 15 | γ-Lactones | 6 |
| Phenols | | 7 | Thiazones | 3 |
| Totals | 176 | 91 | | 132 |

From : Tannenbaum G 2004 Journal of Chemical Education 81: 1131-1135.

Chocolate products are subject to extensive regulation at both supranational and national levels. To provide for a working definition of terms used herein, the norms defined in European Directive 2000/36/EC (2003) are used. A "chocolate" product (also referred to a "dark chocolate" product) is obtained from cocoa products and sugars and contains not less than 25% total cocoa solids (not less than 12% cocoa butter and not less than 14% dry non-fat cocoa solids). It may include up to 5% of vegetable fats other than cocoa butter (from illipe, palm oil, sal, shea, kokum gurgi or mango kernels). "Milk chocolate" contains not less than 25% dry cocoa solids, not less than 14% dry milk solids, not less than 2.5% dry non-fat cocoa solids, not less than 3.5% milk fat, and not less than 25% total fat (cocoa butter and milk fat). "White chocolate" contains not less than 20% cocoa butter and not less than 14% dry milk solids of which not less than 3.5% is milk fat. Chocolate products may also include certain additives, most notably emulsifier lecithin. "Praline" or, when used alone, "chocolate" means a product of single-mouthful size consisting of filled chocolate, whereby the product contains a minimum of 25% chocolate by weight of the product. Such a chocolate or praline may contain up to about 15g of chocolate or compound chocolate mass. Typical pralines weigh between 10g and 15g. "Compound chocolate" product means a chocolate replacement made from a combination of cocoa products, vegetable fat and sweeteners that does not qualify as chocolate products. Typically, such product contains less cocoa material and/or more vegetable fat than chocolate products. It is noted that, in derogation of certain legislation, for the purposes of this application the terms dark chocolate, milk chocolate, white chocolate and compound chocolate are meant to also include products in which sugars normally included, e.g., sucrose and lactose, are supplemented or substituted with other sugars, bulk sweeteners and/or intense sweeteners.

As discussed below, the format of the chocolate or compound chocolate product of the invention is that of a product comprising a casing and a filling. A daily dose of the above-recited functional ingredients, that is vitamin C, vitamin E, zinc, copper and xanthophyll, may be comprised in a single praline or compound chocolate product, or in multiple pralines or compound chocolate products that together provide a daily dose. Other formats that are related to the above preferred format in that they provide a similar barrier function are within the scope of the invention. For example, also encompassed are elongated shapes such as bars or other geometrical shapes containing a core and a casing from which daily portions may be broken off or otherwise removed. Also within the scope of the invention are formats that, in essence, represent multiple pralines (or comparable compound chocolate products) fused into a larger structure, from which individual pralines (or compound chocolate products) may be removed by a consumer. Such forms may take on the appearance of strings on a bead, a series of joined geometrical shapes such as barrels, triangular prisms, etc.

The casing of the praline of the invention may be dark, milk or white chocolate. Preferably, the casing is dark or milk chocolate. The filling may also be any type of chocolate or fat-based confectionary filling. The fat-based filling may have dark, milk or white chocolate flavor. A most preferred fat-based filling is a compound chocolate, containing cocoa powder, sucrose, and milk and vegetable fats. Similar products (i.e., compound chocolate products of the invention) may be made that also have a compound chocolate casing.

As outlined in the summary of chocolate science presented above, eating chocolate is a highly complex mixture of hundreds of chemical compounds, certain of which are highly reactive. Reactions with added chemicals are expected to occur, with the likely result of degradation of chocolate taste, quality and mouthfeel. To applicant's knowledge, no chocolate product containing added vitamins and minerals perceived by customers as a high-quality chocolate product rather than as a "medicinal type" product has been developed to market to date. This absence of high-quality chocolate products containing vitamins and minerals attests to the difficulty in achieving such products. Applicant indeed experienced that direct introduction of vitamin C, vitamin E, lutein/zeaxanthin, zinc salts and copper salts into a single tablet of chocolate results in instability of ingredients as well as in the development of unpleasant taste, after-taste and/or mouthfeel.

For each of the above functional ingredients, the applicant has tested a multitude of commercial preparations presenting ingredients in different formats (i.e., encapsulated or not encapsulated, different chemicals forms, e.g., different salts or esters, encapsulated at different densities and using different matrix materials, etc.) for their suitability for introduction into chocolate. In these experiments ingredients were tested at a concentration representing a daily dose contained in an amount of chocolate considered reasonable for daily consumption (up to about 30 g). Individual products were tested in dark, milk or white chocolate, or fat-based fillings, with or without added flavors such as mint, caramel, orange, cinnamon and other flavors. Subsequently, combinations of different functional ingredients were investigated upon introduction into different chocolate and compound chocolate bases with or without added flavor. Furthermore, experiments were conducted with chocolate products comprising a casing and a filling. Various commercial preparations of the different ingredients were variously included in the casing or the filling of test products. Again, these experiments involved examination of various chocolate or compound bases for casing and filling as well as use of different flavors. Effects of enhancers and astringency blockers were also investigated. The tests performed typically involved a first taste test by independent groups of tasters shortly after production. A product was considered acceptable at this stage, if most testers agreed that the product had the appearance of a fine praline, produced no discernible off-smell, off-taste or after-taste and showed no grittiness or unpleasant mouthfeel. Taste testing was against control products that had the same composition as the test products, except that they did not contain added functional ingredients. Products that were considered acceptable by the latter criteria were subjected to periodic taste retesting and analyses of chemical stability of ingredients using standard methods that are described under Example 1. Continued acceptance of a product required that it continued to satisfy the above criteria of smell, taste and grittiness/mouthfeel and that the functional ingredients remained chemically stable for a period of at least three months.

A solution to the problem of producing a high-quality chocolate or compound chocolate product (i.e., a praline or praline-like product) satisfying the above-detailed taste, texture and stability criteria that contains the desired quantities of functional ingredients vitamin C, vitamin E, zinc, copper and xanthophyll emerged from the latter experimentation. The solution combines the following critical aspects:
(i) The use of a chocolate or compound chocolate format that contains two separate compartments, a casing and a filling, the filling being protected from the environment by the casing;
(ii) Selection of appropriate chemical forms of functional ingredients for introduction in the praline;
(iii) Selective use of encapsulation for certain functional ingredients to be introduced; and
(iv) Appropriate distribution of functional ingredients between casing and filling of the praline.

More specifically, metals are introduced as oxides or salts. Xanthophyll is preferably a mixture containing lutein and a smaller amount of zeaxanthin. No specific requirements have been identified regarding the specific form of vitamin E to be employed. Vitamin C is introduced preferably as an ascorbyl fatty acid ester. Preferably, copper, vitamin E and xanthophylls are used in an encapsulated format. Metals are preferably introduced in the casing, whereas vitamin C, vitamin E and xanthophylls are placed in the filling.

It is noted that the solution identified was able to be practiced over the ranges of concentrations of functional ingredients disclosed herein, i.e., 12-2000 mg of added vitamin C, 2.5-1000 mg added vitamin E (tocopherol equivalents = TE), 1.5-40 mg added zinc, 0.15-10 mg added copper, and 1.5-33 mg added xanthophylls (as a mixture of lutein and zeaxanthin) comprised in two-compartment chocolate(s) or compound chocolate product(s) weighing less than about 30 g. As mentioned before, amounts of ingredients are given are those of the "elemental" or "active" compounds.

The term "encapsulation" as used herein refers to techniques by which one material or a mixture of materials is coated with or entrapped within another material or system. The coated material is called active or core material, and the coating material is called shell, wall material, carrier or encapsulant. Encapsulation techniques are well known to persons skilled in the art. They have been extensively reviewed, e.g., Madene et al 2006 International Journal of Food Science and Technology 41: 1-21; Gibbs et al 1999 International Journal of Food Sciences and Nutrition 50: 213-224. Well known encapsulation processes include chemical techniques such a simple or complex coacervation, liposome entrapment and molecular inclusion as well as mechanical techniques such as spray drying, freeze drying, spray cooling or chilling, extrusion and fluidized bed coating. The different methods tend to yield somewhat different results. For example, particle size may vary, from the low micrometer (e.g., spray-drying) to the millimeter range (e.g., extrusion or fluidized bed coating). Particles may contain different carrier or encapsulant materials, e.g., carbohydrates, gums, proteins, fats, oils, waxes and liposomes. Carbohydrate are extensively utilized as encapsulant. The ability of carbohydrate such as starches, maltodextrins, corn syrup solids and acacia gums to noncovalently bind a variety of different molecules combined with their low cost and diversity make them the preferred choice for an encapsulation support. Granules of some varieties of starch have naturally occurring surface pores of 1-3 µm. New microporous starches were created with the intention of enhancing retention of molecules such as flavors. Golovnya RV et al 1998 Narhung 42: 380-384; Thomas DJ and Atwell WA 1999 Starches. St. Paul, MN; Eagan Press; U.S. Patent No. 5,958,589 to Glenn GM and Stern DJ. Small starch granules spray-dried with small amounts of bonding agents such as protein or water-soluble polysaccharides result in interesting porous structures. Zhao, J. and Whistler, R.J. 1994. Food Technology 48: 104-105. Treating starch granules with amylases will also result in more highly porous structures. Binding of molecules to starch and starch-derived polysaccharides can be either through hydrophobic interactions with the interior of the amylose helix or through surface polar interactions with sugar hydroxyl groups. Maltodextrins are also used in encapsulation, primarily because they are inexpensive, bland in flavor and low-viscosity at high solid ratios. Maltodextrins are formed by partial hydrolysis of cornflower with acids or enzymes and are supplied as dextrose equivalents (DE). DE is a measure of starch polymer hydrolysis. Maltodextrins with different DE are employed for different applications. Bangs WE and Reineccius GA 1981 Journal of Food Science 47: 254-259; Anandaraman S and Reineccius GA 1986 Food Technology 40: 88-93. The most important shortcoming of maltodextrins appears to be their virtual lack of emulsifying capacity. Hydrocolloids such as gums also find use as encapsulating support. Gum arabic is the most often used gum. Its solubility, low viscosity, emulsification properties and ability to retain a variety of compounds make it a versatile encapsulant. The gum is relatively expensive, which is limiting its use. Costs can be reduced somewhat by using blends of gum arabic and maltodextrose. Williams PA and Phillips GO 2000 Gum arabic. In: Handbook of Hydrocolloids (edited by GO Phillips & PA Williams), pp. 155-168. Cambridge. Woodhead Publishing Ltd. Other useful encapsulants are proteins, including sodium caseinate, whey protein and soy protein. Because of their different chemical groups, amphiphilic properties, ability to self-associate and interact with a variety of different substances, large molecular weight and molecular chain flexibility, proteins have excellent functional properties relevant to their use in encapsulation. U.S. Patent No. 5,601,760 to M Rosenberg. Whey proteins are available in the market as whey protein isolates (95-96% protein) or whey protein concentrate (WPC-50, WPC-70) powders. For example, whey protein isolates were found to be a good barrier to oxidation of microencapsulated orange oil. Kim YD and Morr CV 1996 Journal of Agricultural and Food Chemistry 44: 1314-1320. B-lactoglobulin, the most important whey protein, possesses interesting emulsifying and foaming properties and is widely used in food industry. Jouenne E and Crouzet J 2000 Journal of Agricultural and Food Chemistry 48: 1273-1277. Whey proteins have also been used in combination with carbohydrates as carrier proteins. Sheu TY and Rosenberg M 1998 Journal of Food Science 63: 491-494. Sodium caseinate was also found to be an effective wall material for encapsulating orange oil. Kim YD and Morr CV 1996 Caseinate may be even more efficient than whey protein in encapsulating liquid oils. A mixture of casein and carbohydrates such as maltodextrin and corn syrup solid may offer potential as a cost-effective, functional core encapsulating material. Hogan SA et al 2001 Food Engineering and Physical Properties 66 : 675-680. Gelatin is widely used as matrix material, in particular in coacervation. Ducel V et al 2004 Colloids and Surfaces A: Physicochemical Engineering Aspects 232 : 239-247; European Patent Application No. 0 937 496. Other materials used as encapsulants include hydrogenated vegetable oil, stearines, fatty acids, emulsifiers and waxes. Gibbs et al 1999.

Particularly important methods for encapsulation include spray drying. It is widely employed, in particular for encapsulation of flavors. It is also used for dehydration of materials such as powdered milk. Modified starch, maltodextrin, gum or other materials to be used as carrier materials are hydrated. Optimal carrier materials are those that have a high solubility in water, a low viscosity at high concentration, effective emulsification and film-forming properties and efficient drying properties. The active material is homogenized with the carrier material, typically, at a ratio of about 1:4. The mixture is then introduced into a spray dryer and atomized with a nozzle or spinning wheel. Water is evaporated by the hot air contacting the atomized material in the hot chamber. The encapsulated material is then collected after it falls to the bottom of the dryer. The technology has been reviewed by Re-MI 1998 Drying Technology 16: 1195-1236, and Sharma DK and Tiwari BD 2001 Indian Food Industry 20: 48-51. The technique produces relatively small particles in the 10-100 µm range. Often, an agglomeration step is added to increase the size of the particles.

A technique that has been employed primarily in the pharmaceutical and cosmetic industry is fluidized bed coating. Particles to be encapsulated are fluidized in the hot atmosphere of the coating chamber. The encapsulating material is sprayed onto the particles through a nozzle to initiate film formation. The small droplets of the coating solution coalesce on the particle surface. Solvent is evaporated by the hot air, and the encapsulant adheres to the particles. The size of the encapsulated particles grows in the course of repeated wetting and drying stages. Product size varies between about 100 µm and 10 mm. The technique is applicable for hot-melt coatings such as hydrogenated vegetable oil, stearines, fatty acids, emulsifiers and waxes or solvent-based coatings such as starches, gums or maltodextrins. Jacquot M and Pernetti M 2003 In: Cell Immobilization Biotechnology (edited by U Nedovic and R Willaert), pp. 343-356. Series: Focus on biotechnology. Dordrecht: Kluwer Academic Publishers.

Other often-used encapsulation techniques are spray chilling and spray cooling. With spray chilling mixed core and molten carrier material are atomized through a nozzle into a reactor containing chilled air, chilling being typically achieved by means of a carbon dioxide ice bath. Coating droplets adhere to core particle and solidify to form a film. The technique is suitable for encapsulating water-soluble materials that my otherwise be volatilized or damaged during thermal processing. It has been used for encapsulation of vitamins, minerals, bakery products, dry soup mixes, or foods with high fat content. The encapsulating material is frequently a fractionated or hydrogenated vegetable oil. Spray cooling is a related process, the difference lying in the temperature of the air in the reactor. The encapsulant may be a vegetable oil with a melting point in the 45-122°C range. Gouin S 2004 Trends in Food Science and Technology 15: 330-347; Augustin MA et al 2001 Food Australia 53: 220-223; Risch SJ 1995 In: Encapsulation and Controlled Release of Food Ingredient (edited by SJ Risch and GA Reineccius), pp. 2-7. Washington, D.C. American Chemical Society.

Another tested method of encapsulation is extrusion. Gouin S 2004. As a simple example, a volatile compound is dispersed in a matrix polymer at 110°C. The mixture is then forced through a die, and the filaments obtained are plunged into a desiccant liquid that, by hardening the extruded mass, traps the active substances. Rizvi SSH et al 1995 Trends in Food Science and Technology 6: 232-240; Crouzet J 1998 In: Techniques de l'ingénieur, Agroalimentaire F 4100, pp. 1-16, Paris. The liquid most commonly used for hardening and dehydration is isopropyl alcohol. The process has been used, inter alia, for vitamin C and dry food applications.

In principle, any encapsulation method, including the processes described above, can be used for encapsulating functional ingredients to be utilized in the chocolate of chocolate compound products of the invention. The preferred encapsulation method is fluidized bed coating. The method is relatively easy to control and is advantageous economically both in terms of capital and maintenance costs. Furthermore, particle size distribution can be controlled, choice of wall materials is not limited and low porosities can be achieved. Loads (ratio of encapsulated material to matrix) exceeding 50% can be obtained. One apparent disadvantage of using a fluidized bed coating process for coating functional ingredients may be the expected size of the resulting particles. According to the literature, sizes in excess of 100 µm are to be expected. Madene et al. Particle size measurements on some preferred coated ingredients revealed that they all had mean particle sizes greater than 100 µm. Persons skilled in the art of chocolate making know that solids in chocolate must be milled down to a size of maximally 30 µm to avoid a sensation of grittiness. Surprisingly, inclusion of these large particles containing functional ingredients in the nutraceutical chocolates and compound chocolate products of the invention did not produce perceptible grittiness. While not wishing to be bound by any particular theory, it is suggested that the concentration of particles added to achieve the desired ingredient levels may be too low to be sensed.

While vitamin E, xanthophylls lutein and zeaxanthin, copper and zinc may be added to the chocolate or compound chocolate of the invention as separately encapsulated ingredients, it is also possible, for example, to co-encapsulate lutein and zeaxanthin, vitamin E and xanthophylls or zinc and copper prior to introduction into a chocolate product.

Vitamin C can be introduced into the chocolate or compound chocolate of the invention as an ascorbyl ester, in particular as a palmityl, lauryl, cocoyl, oleyl or stearyl ester. The preferred chemical form is that of ascorbyl palmitate.

Vitamin E can be included in the chocolate or compound chocolate of the invention as DL-α-tocopheryl acetate, DL-α-tocopherol, d-α-tocopheryl acetate, d-α-tocopherol or vitamin E isolated from natural sources. Preferably, vitamin E is encapsulated before introduction into the chocolate product.

Copper can be included in a chocolate or compound chocolate of the invention in the form of cupric oxide or cupric salts that include but are not limited to cupric L-alaninate, cupric carbonate, cupric chloride, cupric citrate, cupric gluconate, cupric glycinate, cupric salicylate, cupric sulfate and cupric tartrate. Preferably, the selected copper compound is encapsulated prior to introduction in the chocolate or compound chocolate of the invention.

Zinc can be added as zinc oxide or as a zinc salt including but are not limited to a sulfate, chloride, acetate, gluconate, ascorbate, citrate, aspartate, picolinate, orotate and transferrin salt. The selected zinc compound may or may not be encapsulated prior to introduction in the chocolate or compound chocolate of the invention.

Functional ingredients in encapsulated or un-encapsulated form suitable for use in the products of the invention can be obtained from Cognis (Germany), Fortitech Inc. (United States), Lithos Food (Netherlands), LycoRed Ltd. (Israel), Sigma Aldrich (United States), or Royal DSM N.V. (Netherlands). Aromas, extracts and flavors can be sourced from Diana Naturals (France), Fabbri 1905 SpA (Italy), or A.M. Todd Company (United States). Couverture chocolate, compound coatings and fillings can be procured from Barry Callebaut AG (Switzerland), Luebecker Marzipan Fabrik v. Minden & Bruhns GmbH & Co. KG (Germany), Max Felchlin AG (Switzerland), or Valrhona (France).

The filling of a chocolate or compound chocolate of the invention may optionally comprise a flavor such as, for example, mint, orange, spicy orange, caramel, butter scotch, vanilla, coffee, cinnamon, pepper, chili, raspberry, strawberry, ginger, hazelnut, honey, lemon, lime, papaya, pistachio, pecan, tamarind, tangerine or walnut.

A chocolate or compound chocolate of the invention may be manufactured by essentially the same methods that are used to prepare conventional chocolates or pralines (and analogous compound chocolate products). The only difference lies in the inclusion of the functional ingredients. Preferably, the functional ingredients are introduced into casing or filling masses subsequent to all milling steps, the principal reason being that some of the ingredients should not be exposed to elevated temperatures for extended periods of time. As an additional reason, if ingredients encapsulated by a fluidized bed coating process are employed, addition of these encapsulated ingredients during a milling step would destroy the capsules and expose the encapsulated ingredient. Ideal stages for introduction of ingredients are the final tempering stages. It has been noted that, at the higher end of concentration ranges, addition of ascorbyl fatty acid ester, encapsulated xanthophylls and encapsulated vitamin E increases the viscosity of the filling material. This effect may be corrected by addition of an appropriate quantity of vegetable or milk fat to the filling material.

The pralines or compound chocolates of the invention also include varieties in which sucrose, the sugar normally present in chocolate-type products, or lactose present in milk chocolate products, is supplemented with or substituted in part or in whole with other carbohydrates, bulk sweeteners or intense sweeteners. Foods with low glycemic effects (blood sugar raising ability) have been scientifically validated as having an important role in the dietary management of weight reduction, diabetic and reducing the risk of heart disease and hypertension. Leeds A et al 1998 American Journal of Clinical Nutrition 62 (Suppl) : 1161S-1168S. To produce pralines or compound chocolates of the invention that have a reduced glycemic effect, the sugar in the chocolate or compound chocolate base utilized for their manufacture can be replaced in part or in whole with other carbohydrates, bulk sweeteners or intense sweeteners, either used alone or in various combinations. The person skilled in the art of chocolate making will know if and how processes of manufacture need to altered to produce quality low glycemic index chocolate or compound chocolate products containing alternative carbohydrates, bulk sweeteners and/or intense sweeteners.

Typical alternative carbohydrates include but are not limited to fructose, tagatose, lactose, isomaltulose, trehalose, polydextrose and inulin. Typical bulk sweeteners or sugar alcohols include but are not limited to sorbitol, mannitol, xylitol, erythritol, maltitol, isomalt (a 1:1 combination of two disaccharide alcohols) and lactitol. Intense sweeteners include but are not limited to Acesulfamine potassium, Alitame, Aspartame, Aspartate-acesulfame salt, Cyclamate, Neotame, Saccharin, Stevia, Sucralose and Thaumatin. It is noted that intense sweeteners are also being referred to as "artificial sweeteners" even though they include natural plant extracts such as stevia glycosides from the stevia plant or Thaumatin, a sweet protein from a West African plant.

Alternative carbohydrates, bulk sweeteners and intense sweeteners are often used in combination to make low glycemic index chocolate. For example, diabetic chocolates are being made with polydextrose and/or disaccharide alcohols (e.g., maltitol or lactitol) and sweetened up with intense sweeteners (w.g., Aspartame or Sucalose). To correct the somewhat flat sweetness of such products, low levels of monosaccharide sugars or alcohols can be added (e.g., 6% fructose, 5% xylitol or 4% erythritol). It is noted that the level of monosaccharide sugars and alcohols is typically kept at a low level, because when used at elevated concentrations these compounds produce a somewhat scratchy, burning after-taste. Also, high levels of sugar alcohol have an undesired laxative effect.

The present invention also relates to a method for treatment or prevention of diseases of the eye, in particular of macular degeneration and cataract formation, or for maintaining or enhancing general eye heath, which method entails daily administration to a subject in need of such therapy a daily dose of chocolate or compound chocolate product of the invention. This therapy can be expanded by the co-administration of another nutritional supplement, e.g., a supplement comprising omega-3 fatty acids. Omega-3 fatty acids are also considered as being beneficial to eye health. The term "co-administration" is understood to mean administration of supplement on the same day the chocolate or compound chocolate product of the invention is administered, whereby the supplement may be ingested at the same time as, before or subsequent to the chocolate or compound chocolate product of the invention.

It is also envisaged that subjects in need of preventative or actual therapy of diseases of the eye such as macular degeneration or cataract formation or other consumers may wish to alternate between conventional supplements in pill or gel cap form and the chocolate or compound chocolate product of the invention. Hence, therapies in which a subject switches between conventional supplements and the product of the invention are also considered to relate to the present invention, as long as the product of the invention forms a regular part of the subject's regimen, defined herein as at least one dose of product of the invention per week of therapy.

In the above-mentioned methods of preventative or actual therapy of eye disease, the chocolate or compound chocolate product of the invention is used as a medicament for the treatment or prevention of diseases of the eye such as macular degeneration or cataract formation.

Finally, also encompassed by the present invention are kits that comprise chocolates or chocolate compound products of the invention in numbers sufficient for weekly, biweekly or monthly therapy or general health use.

These kits would comprise chocolates or compound chocolate products presented in kit form, the kit comprising packaging which would inform the consumer of the status of his therapy at any point in the time course of that therapy. This might comprise, for example, package design, involving double or triple packaging, labeling and illustrative leaflets indicating day, week and month of the intended chocolate consumption inducing the wanted intake and may involve repetitious and redundant labeling, physical barriers limiting access to certain chocolate pieces destined for future consumption, and other methods which would remind and witness the desired consumption kinetics and therefore attempt to provide therapeutic compliance via a kit format.

### Examples

### Example 1: Analytical methods

Vitamin E: The chocolate sample (the separated shell or filling of a praline) undergoes saponification under an inert atmosphere. The analyte is extracted with diethyl ether and evaporated to dryness with nitrogen. The redissolved extract is analyzed using RP-HPLC and fluorescence detection. Quantification of the analyte is performed using an external analytical standard of DL-α-tocopherol. Saponification and extraction steps are controlled by performing a recovery experiment.

Vitamin C: The analyte is extracted from the chocolate sample (the separated shell or filling of a praline) with dioxane/methanol/phosphoric acid and analyzed using RP-HPLC and UV detection. The quantification of the analyte is performed using an external analytical standard of ascorbyl palmitate, and amount of free ascorbic acid is calculated. The extraction step is controlled by performing a recovery experiment.

Lutein/Zeaxanthin: The chocolate sample (the separated shell or filling of a praline) undergoes saponification under an inert atmosphere. The analyte is extracted with diethyl ether and evaporated to dryness with nitrogen. The redissolved extract is analyzed using RP-HPLC and UV detection. Quantification of the analyte is performed using an external standard method, using a lutein/zeaxanthin raw material as reference material. Alternatively, independent analytical standards for lutein and zeaxanthin are used.

Zinc/Copper: Water, nitric acid and hydrogen peroxide are added to the chocolate sample (the separated shell or filling of a praline). The suspension is digested under pressure in a microwave oven. The digestion fluid is transferred to a 10ml tube and filled up to volume with pure water. The solution is analyzed by inductively coupled plasma optical emission spectrometry (ICP-OES). Quantification of the analyte is performed using an external analytical standard for copper and zinc (multistandard).

### Example 2: Method of preparation of example chocolates or compound chocolate products of the invention

1. Preparation of the casing of a product of the invention:
   (a) an appropriate amount of chocolate base (see the subsequent examples) is melted at about 40°C,
   (b) while the chocolate mass is being stirred with a food mixer, functional ingredients are added one after the other over a period of about 2 minutes, avoiding the formation of clumps,
   (c) the supplemented chocolate mass is tempered manually for about 5 minutes at about 31 °C,
   (d) a large fraction of the mass is poured into a mold (e.g., a mold having 24 cavities) at 28-30°C, all cavities being completely filled and excess chocolate removed, whereas a small fraction (used for closing the chocolates) is kept in a wheel machine at 31 °C to 33°C,
   (e) the mold is vibrated using a vibrating table for about 60 seconds,
   (f) the mold is turned upside down to allow excess chocolate to drain,
   (g) the mold is weighed to determine the weight of the chocolate cups; if needed, the draining process is continued,
   (h) the mold with cavities facing down is vibrated for 3 to 5 seconds to optimize shape and homogenous distribution of chocolate in the cavities,
   (i) the mold is righted and allowed to cool for 3 to 5 minutes for hardening of the chocolate cups, and
   (j) the mold is placed in a refrigerator at 12°C for about 20 minutes.
2. Preparation of the filling of a product of the invention:
   (a) filling chocolate is heated to 40° C,
   (b) while the filling mass is being stirred with a food mixer, functional ingredients are added one after the other over a period of about 5 minutes, avoiding the formation of clumps, and
   (c) the supplemented filling mass is tempered manually for about 5 minutes at about 30°C.
3. Filling of chocolate cups:
   (a) the filling mass of step 2 (at 30°C) is put into a piping bag,
   (b) the mold is placed on a scale,
   (c) filling mass is added to each cup, the correct amount of filling added to each cup being estimated based on increased mold weight, and
   (d) the mold is placed in a refrigerator at 10°C for about 30 minutes.
4. Closure of the chocolate cups:
   (a) the mold is taken out of the refrigerator, and heat is applied to the open chocolate cups to soften their rims,
   (b) the mold is placed under the curtain of the wheel machine (chocolate "waterfall") containing liquid supplemented chocolate mass (see step 1d),
   (c) the filled mold is placed on a scale, and excess chocolate is removed as needed to remain within indicated weight limits,
   (d) the mold is subsequently placed in a refrigerator at 10°C for about 20 minutes, and
   (e) the mold is inverted to release the finished chocolates.
5. Finished chocolates are placed in a silver foil bag that is sealed (without applying a vacuum).
6. Packaged chocolates are stored in a cool and dry place (about 19°C; humidity at about 60%).

The above process description illustrates the general procedure followed; the procedure is adapted slightly to accommodate differences in the characteristics of the chocolate or compound chocolate bases, fats, and functional ingredients used.

The following examples present recipes for chocolates and compound chocolate products of the invention. The recipes are for batches of 10 kg. From such batches 760 chocolates or products may be made, each having a weight of about 13 g.

### Example 3: Chocolate of the invention, version A

| | | | Grams |
|---|---|---|---|
| **Casing** | | | |
| | **Substrate** | | |
| | | Cocoa mass | 2438.3 |
| | | Cocoa butter | 124.0 |
| | | Vegetable fat | 34.0 |
| | | Sugar | 1549.8 |
| | | Soy lecithin | 20.7 |
| | | | |

| | **Functional ingredients** | | |
|---|---|---|---|
| | | Encapsulated zinc oxide (50%) | 24.8 |
| | | Encapsulated copper gluconate (50%) | 9.1 |
| | | | |

| **Filling** | | | |
|---|---|---|---|
| | **Substrate** | | |
| | | Cocoa powder | 188.1 |
| | | Vegetable fat | 2505.5 |
| | | Sugar | 1354.0 |
| | | Milk sugar | 357.3 |
| | | Skimmed milk powder | 357.3 |
| | | Soy lecithin | 18.8 |
| | | | |
| | **Flavor** | Natural mint extract | 11.6 |
| | | | |

| | **Functional ingredients** | | |
|---|---|---|---|
| | | Encapsulated dl-alpha-tocopheryl acetate (50%) | 380.0 |
| | | Encapsulated lutein (5%) (contains zeaxanthin) | |
| | | | 114.0 |
| | | Vitamin C palmitate | 514.2 |

### Example 4: Chocolate of the invention, version B

| | | | | |
|---|---|---|---|---|
| | | | Grams | |

| **Casing** | | | | |
|---|---|---|---|---|
| | **Substrate** | | | |
| | | Cocoa mass | | 2453.2 |
| | | Cocoa butter | | 124.7 |
| | | Vegetable fat | | 21.3 |
| | | Sugar | | 1559.3 |
| | | Soy lecithin | | 20.8 |
| | | | | |
| | **Functional ingredients** | | | |
| | | Encapsulated zinc oxide (50%) | | 9.9 |
| | | Encapsulated copper gluconate (50%) | | 11.4 |
| | | | | |

| **Filling** | | | | |
|---|---|---|---|---|
| | **Substrate** | | | |
| | | Cocoa powder | | 235.0 |
| | | Vegetable fat | | 2407.0 |
| | | Sugar | | 1691.9 |
| | | Milk sugar | | 446.5 |
| | | Skimmed milk powder | | 446.5 |
| | | Soy lecithin | | 23.5 |
| | | | | |
| | **Flavor** | Natural mint extract | | 11.6 |
| | | | | |
| | **Functional ingredients** | | | |
| | | Encapsulated dl-alpha-tocopheryl acetate (50%) | | 54.7 |
| | | Encapsulated lutein (5%) (contains zeaxanthin) | | 114.0 |
| | | Vitamin C palmitate | | 370.3 |

### Example 5: Chocolate of the invention, version C

| | | | | |
|---|---|---|---|---|
| | | | Grams | |

| **Casing** | | | | |
|---|---|---|---|---|
| | **Substrate** | | | |
| | | Cocoa powder | | 544.5 |
| | | Vegetable fat | | 1346.4 |
| | | Sugar | | 2282.6 |
| | | Soy lecithin | | 20.9 |
| | | | | |
| | **Functional ingredients** | | | |
| | | Encapsulated zinc oxide (50%) | | 6.2 |
| | | | | |

| **Filling** | | | | |
|---|---|---|---|---|
| | **Substrate** | | | |
| | | Vegetable fat | | 2526.2 |
| | | Sugar | | 1328.8 |
| | | Milk sugar | | 393.0 |
| | | Skimmed milk powder | | 486.6 |
| | | Soy lecithin | | 37.4 |
| | | | | |
| | **Flavor** | Natural orange extract | | 11.6 |
| | **Functional ingredients** | | | |
| | | Encapsulated dl-alpha-tocopheryl acetate (50%) | | 380.0 |
| | | Encapsulated lutein (5%) (contains zeaxanthin) | | 114.0 |
| | | Vitamin C palmitate | | 514.2 |
| | | Encapsulated copper gluconate (50%) | | 9.1 |

### Example 6: Chocolate of the invention, version D

| | | | | |
|---|---|---|---|---|
| | | | Grams | |

| **Casing** | | | | |
|---|---|---|---|---|
| | **Substrate** | | | |
| | | Cocoa mass | | 2358.2 |
| | | Cocoa butter | | 119.9 |
| | | Vegetable fat | | 101.9 |
| | | Sugar | | 1498.8 |
| | | Soy lecithin | | 20.0 |
| | | | | |
| | **Functional ingredients** | | | |
| | | Encapsulated zinc oxide (50%) | | 74.5 |
| | | Encapsulated copper gluconate (50%) | | 27.4 |
| | | | | |

| **Filling** | | | | |
|---|---|---|---|---|
| | **Substrate** | | | |
| | | Cocoa powder | | 55.0 |
| | | Vegetable fat | | 2785.0 |
| | | Sugar | | 395.9 |
| | | Milk sugar | | 104.5 |
| | | Skimmed milk powder | | 104.5 |
| | | Soy lecithin | | 5.5 |
| | | | | |
| | **Flavor** | Natural mint extract | | 11.6 |
| | **Functional ingredients** | | | |
| | | Encapsulated dl-alpha-tocopheryl acetate (50%) | | 912.0 |
| | | Encapsulated lutein (20%) (contains zeaxanthin) | | 85.5 |
| | | Vitamin C palmitate | | 1341.5 |

### Example 7: Chocolate of the invention, version E

| | | | | |
|---|---|---|---|---|
| | | | Grams | |

| **Casing** | | | | |
|---|---|---|---|---|
| | **Substrate** | | | |
| | | Cocoa mass | | 2226.6 |
| | | Cocoa butter | | 113.2 |
| | | Butter oil | | 213.4 |
| | | Sugar | | 1415.2 |
| | | Soy lecithin | | 18.9 |
| | | | | |
| | **Functional ingredients** | | | |
| | | Zinc gluconate | | 208.6 |
| | | Encapsulated copper oxide (50%) | | 4.8 |
| | | | | |

| **Filling** | | | | |
|---|---|---|---|---|
| | **Substrate** | | | |
| | | Cocoa powder | | 53.8 |
| | | Vegetable fat | | 2787.4 |
| | | Sugar | | 387.5 |
| | | Milk sugar | | 102.3 |
| | | Skimmed milk powder | | 102.3 |
| | | Soy lecithin | | 5.4 |
| | | | | |
| | **Flavor** | Natural mint extract | | 23.2 |
| | | | | |
| | **Functional ingredients** | | | |
| | | Encapsulated dl-alpha-tocopheryl acetate (50%) | | 912.0 |
| | | Encapsulated lutein (20%) (contains zeaxanthin) | | 85.5 |
| | | Vitamin C palmitate | | 1341.5 |

### Example 8: Chocolate of the invention, version F

| **Casing** | | | Grams | |
|---|---|---|---|---|
| | **Substrate** | | | |
| | | Cocoa powder | | 534.9 |
| | | Vegetable fat | | 1359.7 |
| | | Sugar | | 2242.4 |
| | | Soy lecithin | | 20.6 |
| | | | | |
| | **Functional ingredients** | | | |
| | | Zinc oxide | | 24.8 |
| | | Encapsulated copper gluconate (50%) | | 18.2 |
| | | | | |

| **Filling** | | | | |
|---|---|---|---|---|
| | **Substrate** | | | |
| | | Vegetable fat | | 2671.1 |
| | | Sugar | | 814.3 |
| | | Milk sugar | | 240.8 |
| | | Skimmed milk powder | | 298.2 |
| | | Soy lecithin | | 22.9 |
| | | | | |
| | **Flavor** | Caramel syrup | | 23.2 |
| | | | | |
| | **Functional ingredients** | | | |
| | | Encapsulated dl-alpha-tocopheryl acetate (50%) | | 608.0 |
| | | Encapsulated lutein (5%) (contains zeaxanthin) | | 228.0 |
| | | Vitamin C palmitate | | 894.3 |

### Example 9: Chocolate (Comparative)

| | | | |
|---|---|---|---|
| | | | Grams |

| **Casing** | | | |
|---|---|---|---|
| | Substrate | | |
| | | Cocoa mass | 1869.3 |
| | | Cocoa butter | 483.1 |
| | | Maltitol | 1827.3 |
| | | Soy lecithin | 21.0 |
| | | | |

| **Filling** | | | |
|---|---|---|---|
| | Substrate | | |
| | | Cocoa powder | 202.5 |
| | | Vegetable fat | 2513.9 |
| | | Whey powder | 349.8 |
| | | Skimmed milk powder | 349.8 |
| | | Maltitol | 1307.0 |
| | | Soy lecithin | 18.4 |
| | | | |
| | **Flavor** | Natural orange extract | 17.4 |
| | | | |
| | **Functional ingredients** | Encapsulated zinc oxide (50%) | 24.8 |
| | | Encapsulated copper gluconate (50%) | 9.1 |
| | | Encapsulated dl-alpha-tocopheryl acetate (50%) | 380.0 |
| | | Encapsulated lutein (5%) (contains zeaxanthin) | 114.0 |
| | | | |
| | | Vitamin C palmitate | 514.2 |

### Example 10: Chocolate of the invention, version H

| | | | | |
|---|---|---|---|---|
| | | | Grams | |

| **Casing** | | | | |
|---|---|---|---|---|
| | **Substrate** | | | |
| | | Cocoa mass | | 2399.7 |
| | | Cocoa butter | | 122.0 |
| | | Vegetable fat | | 66.7 |
| | | Sugar | | 1525.2 |
| | | Soy lecithin | | 20.3 |
| | | | | |
| | **Functional ingredients** | | | |
| | | Zinc oxide | | 31.9 |
| | | Encapsulated copper gluconate (50%) | | 34.7 |
| | | | | |

| **Filling** | | | | |
|---|---|---|---|---|
| | **Substrate** | | | |
| | | Cocoa powder | | 10.91 |
| | | Vegetable fat | | 2877.6 |
| | | Sugar | | 78.5 |
| | | Milk sugar | | 20.7 |
| | | Skimmed milk powder | | 20.7 |
| | | Soy lecithin | | 1.1 |
| | | | | |
| | **Flavor** | Natural cinnamon extract | | 17.4 |
| | **Functional ingredients** | | | |
| | | Encapsulated dl-alpha-tocopheryl acetate (50%) | | 894.1 |
| | | Encapsulated lutein (5%) (contains zeaxanthin) | | 91.2 |
| | | | | |
| | | Vitamin C palmitate | | 1788.7 |

### Example 11: Chocolate of the invention, version I

| **Casing** | | | Grams |
|---|---|---|---|
| | **Substrate** | | |
| | | Cocoa mass | 2310.6 |
| | | Cocoa butter | 117.5 |
| | | Butter oil | 142.2 |
| | | Sugar | 1468.6 |
| | | Soy lecithin | 19.6 |
| | | | |
| | **Functional ingredients** | | |
| | | Zinc gluconate | 139.1 |
| | | Encapsulated copper oxide (50%) | 3.2 |
| | | | |

| **Filling** | | | |
|---|---|---|---|
| | **Substrate** | | |
| | | Cocoa powder | 121.3 |
| | | Vegetable fat | 2645.8 |
| | | Sugar | 873.3 |
| | | Milk sugar | 230.5 |
| | | Skimmed milk powder | 230.5 |
| | | Soy lecithin | 12.1 |
| | | | |
| | **Flavor** | Natural mint extract | 23.2 |
| | | | |
| | **Functional ingredients** | | |
| | | | |
| | | Encapsulated DL-α-tocopherol (50%) Encapsulated lutein (20%) (contains | 509.2 |
| | | zeaxanthin) | 57.0 |
| | | L-ascorbyl-6-O-stearate | 1098.1 |

### Example 12: Chocolate of the invention, version J

| | | | | |
|---|---|---|---|---|
| | | | Grams | |

| **Casing** | | | | |
|---|---|---|---|---|
| | **Substrate** | | | |
| | | Cocoa mass | | 2498.3 |
| | | Cocoa butter | | 122.0 |
| | | Vegetable fat | | 67.9 |
| | | Sugar | | 1524.3 |
| | | Soy lecithin | | 20.3 |
| | | | | |
| | **Functional ingredients** | | | |
| | | Encapsulated zinc oxide (50%) | | 49.7 |
| | | Encapsulated copper gluconate (50%) | | 18.2 |
| | | | | |

| **Filling** | | | | |
|---|---|---|---|---|
| | **Substrate** | | | |
| | | Cocoa powder | | 110.0 |
| | | Vegetable fat | | 2669.4 |
| | | Sugar | | 792.3 |
| | | Milk sugar | | 209.1 |
| | | Skimmed milk powder | | 209.1 |
| | | Soy lecithin | | 11.0 |
| | | | | |
| | **Flavor** | Natural mint extract | | 11.6 |
| | | | | |
| | **Functional ingredients** | | | |
| | | Encapsulated dl-alpha-tocopheryl acetate (50%) | | 760.0 |
| | | Vitamin C palmitate | | 1028.5 |

## Claims

1. A chocolate or compound chocolate product comprising added functional ingredients including vitamin C, vitamin E, zinc, copper and, optionally, a xanthophyll, the chocolate or chocolate compound product having a casing and a filling wherein at least the vitamin C, the vitamin E and the xanthophyll are comprised in the filling and wherein one or both of the zinc and the copper are present in the casing, and the ingredients are essentially chemically stable and do not produce a textural or taste effect detectable by a typical consumer during the shelf life of the chocolate or compound chocolate product, provided that the concentration of the added vitamin C does not exceed about 75 mg/g, that of the added vitamin E about 45 mg/g, that of the added zinc about 3 mg/g, that of the added copper about 0.4 mg/g and that of the added xanthophyll about 2.6 mg/g of the chocolate or compound chocolate product.

2. The chocolate or compound chocolate product of claim 1, wherein the vitamin C is added as a palmityl, lauryl, cocoyl, oleyl or stearyl ester.

3. The chocolate or compound chocolate product of claim 1 or 2, wherein the vitamin C is added as a palmityl ester.

4. The chocolate or compound chocolate product of any one of the preceding claims, wherein the optionally added xanthophyll is lutein or a combination of lutein and zeaxanthin.

5. The chocolate or compound chocolate product of any one of the preceding claims, wherein the vitamin E, the copper and, if included, the xanthophyll are added as encapsulated ingredients.

6. The chocolate or compound chocolate product of claim 5, wherein the zinc is also added as an encapsulated ingredient.

7. The chocolate or compound chocolate product of claim 5 or 6 wherein the mean particle size of encapsulated ingredients is greater than about 100 µm.

8. The chocolate or compound chocolate product of claim 5 or 6 wherein copper and zinc, or xanthophyll and vitamin E are added subsequent to co-encapsulation.

9. The chocolate or compound chocolate product of any one of the preceding claims, wherein:
a) zinc is added as encapsulated or non-encapsulated zinc oxide or zinc salt in the form of a sulfate, chloride, acetate, gluconate, ascorbate, citrate, aspartate, picolinate, orotate or transferrin salt;
b) copper is added in the form of encapsulated cupric oxide or cupric salt including cupric L-alaninate, cupric carbonate, cupric chloride, cupric citrate, cupric gluconate, cupric glycinate, cupric oxide, cupric salicylate, cupric sulfate or cupric tartrate; and
c) vitamin E is added in the form of encapsulated DL-α-tocopheryl acetate, DL-α-tocopherol, d-α-tocopheryl acetate, d-α-tocopherol or vitamin E isolated from natural sources.

10. The chocolate or compound chocolate product of any one of the preceding claims, wherein a normal sugar constituent such as sucrose or lactose contained in the casing or the filling is supplemented or substituted in whole or in part by one or more of an alternative carbohydrate such as one or more compounds selected from the group consisting of fructose, tagatose, lactose, isomaltose, trehalose, polydextrose and inulin; a bulk sweetener such as one or more compounds selected from the group consisting of sorbitol, mannitol, xylitol, erythritol, maltitol, isomalt and lactitol; and an intense sweetener such as one or more compounds selected from the group consisting of Acesulfamine potassium, Alitame, Aspartame, Aspartate-acesulfame salt, Cyclamate, Neotame, Saccharin, Stevia, Sucralose and Thaumatin.

11. A single chocolate or compound chocolate product or a plurality of chocolates or compound chocolate products according to any one of the preceding claims, intended for daily consumption, comprising:
a) 12-2000 mg of added vitamin C, 2.5-1000 mg added vitamin E, 1.5-40 mg added zinc, 0.15-10 mg added copper and, optionally, 1.5-33 mg added xanthophyll; or
b) 180-1000 mg of added vitamin C, 30-500 mg added vitamin E, 5-30 mg added zinc, 1-2 mg added copper and 7-20 mg added xanthophyll; or
c) 500 mg of added vitamin C, 294 mg added vitamin E, 25 mg added zinc, 1.6 mg added copper and 12 mg added xanthophyll.

12. A method of producing the chocolate or compound chocolate product of any one of the preceding claims, comprising making a chocolate or compound chocolate product and the further step of adding appropriate amounts of functional ingredients vitamin C, vitamin E, zinc, copper and, optionally, xanthophyll to liquid chocolate or compound chocolate masses used for preparing casing and filling, whereby the additional step occurs subsequent to all milling steps.

13. A single chocolate or compound chocolate product or the plurality of chocolates or compound chocolate products of any one of claims 1 to 11, for use in the dietetic prevention and/or treatment of diseases of the eye, or for supporting general eye health, wherein the single chocolate or compound chocolate product or the plurality of chocolates or compound chocolate products are for daily administration to a subject.

14. The product(s) of claim 13, wherein the disease that is treated or prevented is age-related macular degeneration or cataract formation.

15. The product(s) of claim 13, wherein the single chocolate or compound chocolate product or the plurality of chocolates or compound chocolate products according to any one of claims 1 to 11 is co-administered with another nutritional supplement, such as a nutritional supplement that provides omega-3 fatty acids.

## Patentansprüche

1. Schokolade oder Schokoladezusammensetzungsprodukt, umfassend zugesetzte funktionelle Bestandteile einschließlich Vitamin C, Vitamin E, Zink, Kupfer und gegebenenfalls ein Xanthophyll, wobei die Schokolade oder das Schokoladezusammensetzungsprodukt eine Hülle und eine Fülle aufweist, wobei zumindest das Vitamin C, das Vitamin E und das Xanthophyll in der Fülle vorliegen und wobei eines oder beide von Zink und Kupfer in der Hülle vorliegen, und wobei die Bestandteile im Wesentlichen chemisch stabil sind und zu keinem von einem typischen Konsumenten während der Haltbarkeitsdauer der Schokolade oder des Schokoladezusammensetzungsprodukts wahrnehmbaren Textur- oder Geschmackseffekt führen, mit der Maßgabe, dass die Konzentration an zugesetztem Vitamin C ungefähr 75 mg/g, diejenige des zugesetzten Vitamins E ungefähr 45 mg/g, diejenige des zugesetzten Zinks ungefähr 3 mg/g, diejenige des zugesetzten Kupfers ungefähr 0,4 mg/g und diejenige des zugesetzten Xanthophylls ungefähr 2,6 mg/g der Schokolade oder des Schokoladezusammensetzungsprodukts nicht überschreitet.

2. Schokolade oder Schokoladezusammensetzungsprodukt nach Anspruch 1, wobei das Vitamin C als Palmityl-, Lauryl-, Cocoyl-, Oleyl- oder Stearylester zugesetzt wird.

3. Schokolade oder Schokoladezusammensetzungsprodukt nach Anspruch 1 oder 2, wobei das Vitamin C als Palmitylester zugesetzt wird.

4. Schokolade oder Schokoladezusammensetzungsprodukt nach einem der vorhergehenden Ansprüche, wobei es sich bei dem gegebenenfalls zugesetzten Xanthophyll um Lutein oder eine Kombination von Lutein und Zeaxanthin handelt.

5. Schokolade oder Schokoladezusammensetzungsprodukt nach einem der vorhergehenden Ansprüche, wobei das Vitamin E, das Kupfer und, falls mitverwendet, das Xanthophyll als verkapselte Bestandteile zugesetzt werden.

6. Schokolade oder Schokoladezusammensetzungsprodukt nach Anspruch 5, wobei das Zink ebenfalls als verkapselter Bestandteil zugesetzt wird.

7. Schokolade oder Schokoladezusammensetzungsprodukt nach Anspruch 5 oder 6, wobei die mittlere Teilchengröße der verkapselten Bestandteile größer als ungefähr 100 µm ist.

8. Schokolade oder Schokoladezusammensetzungsprodukt nach Anspruch 5 oder 6, wobei Kupfer und Zink oder Xanthophyll und Vitamin E, nach der gemeinsamen Verkapselung zugesetzt werden.

9. Schokolade oder Schokoladezusammensetzungsprodukt nach einem der vorhergehenden Ansprüche, wobei:
a) Zink als verkapseltes oder nichtverkapseltes Zinkoxid oder Zinksalz in Form eines Sulfats, Chlorids, Acetats, Gluconats, Ascorbats, Citrats, Aspartats, Picolinats, Orotats oder Transferrinsalzes zugesetzt wird;
b) Kupfer in Form von verkapseltem Kupfer(II)-Oxid oder Kupfer(II)-Salz einschließlich Kupfer(II)-L-Alaninat, Kupfer(II)-Carbonat, Kupfer(II)-Chlorid, Kupfer(II)-Citrat, Kupfer(II)-Gluconat, Kupfer(II)-Glycinat, Kupfer(II)-Oxid, Kupfer(II)-Salicylat, Kupfer(II)-Sulfat oder Kupfer(II)-Tartrat zugesetzt wird; und
c) Vitamin E in Form von verkapseltem DL-α-Tocopherylacetat, DL-α-Tocopherol, d-α-Tocopherylacetat, d-α-Tocopherol oder Vitamin E, das aus natürlichen Quellen isoliert wurde, zugesetzt wird.

10. Schokolade oder Schokoladezusammensetzungsprodukt nach einem der vorhergehenden Ansprüche, wobei ein normaler Zuckerbestandteil wie Saccharose oder Lactose, der in der Hülle oder in der Fülle enthalten ist, ganz oder teilweise durch eines oder mehrere eines alternativen Kohlenhydrats ergänzt oder substituiert ist, wie einer oder mehreren Verbindungen, ausgewählt aus der Gruppe bestehend aus Fructose, Tagatose, Lactose, Isomaltose, Trehalose, Polydextrose und Inulin; einem Füllsüßungsmittel, wie einer oder mehreren Verbindungen, ausgewählt aus der Gruppe bestehend aus Sorbit, Mannit, Xylit, Erythrit, Maltit, Isomalt und Lactit; und einem Süßstoff wie eine oder mehrere Verbindungen, ausgewählt aus der Gruppe bestehend aus Acesulfamin-Kalium, Alitam, Aspartam, Aspartat-Acesulfamsalz, Cyclamat, Neotam, Saccharin, Stevia, Sucralose und Thaumatin.

11. Einzelne Schokolade oder einzelnes Schokoladezusammensetzungsprodukt oder Mehrzahl von Schokoladen oder Schokoladezusammensetzungsprodukten nach einem der vorhergehenden Ansprüche, das für den täglichen Verzehr bestimmt ist, umfassend:
a) 12-2000 mg zugesetztes Vitamin C, 2,5-1000 mg zugesetztes Vitamin E, 1,5-40 mg zugesetztes Zink, 0,15-10 mg zugesetztes Kupfer und gegebenenfalls 1,5-33 mg zugesetztes Xanthophyll; oder
b) 180-1000 mg zugesetztes Vitamin C, 30-500 mg zugesetztes Vitamin E, 5-30 mg zugesetztes Zink, 1-2 mg zugesetztes Kupfer und 7-20 mg zugesetztes Xanthophyll; oder
c) 500 mg zugesetztes Vitamin C, 294 mg zugesetztes Vitamin E, 25 mg zugesetztes Zink, 1,6 mg zugesetztes Kupfer und 12 mg zugesetztes Xanthophyll.

12. Verfahren zur Herstellung der Schokolade oder des Schokoladezusammensetzungsprodukts nach einem der vorhergehenden Ansprüche, umfassend: Herstellen einer Schokolade oder eines Schokoladezusammensetzungsprodukts und den weiteren Schritt: Zusetzen von geeigneten Mengen der funktionellen Bestandteile Vitamin C, Vitamin E, Zink, Kupfer und gegebenenfalls Xanthophyll zu flüssigen Schokolade- oder Schokoladezusammensetzungsmassen, die für die Herstellung von Hülle und Fülle verwendet werden, wobei der zusätzliche Schritt nach allen Mahlschritten erfolgt.

13. Einzelne Schokolade oder einzelnes Schokoladezusammensetzungsprodukt oder Mehrzahl von Schokoladen oder Schokoladezusammensetzungsprodukten nach einem der Ansprüche 1 bis 11 zur Verwendung in der diätetischen Vorbeugung und/oder Behandlung von Augenkrankheiten oder zum Unterstützen der allgemeinen Augengesundheit, wobei die einzelne Schokolade oder das einzelne Schokoladezusammensetzungsprodukt oder die Mehrzahl von Schokoladen oder Schokoladezusammensetzungsprodukten für die tägliche Verabreichung an ein Individuum bestimmt sind.

14. Produkt(e) nach Anspruch 13, wobei es sich bei der Krankheit, die behandelt wird, bzw. der vorgebeugt wird, um altersbedingte Makuladegeneration oder Kataraktbildung handelt.

15. Produkt(e) nach Anspruch 13, wobei die einzelne Schokolade oder das einzelne Schokoladezusammensetzungsprodukt oder die Mehrzahl von Schokoladen oder Schokoladenzusammensetzungsprodukten nach einem der Ansprüche 1 bis 11 gemeinsam mit einem weiteren Nahrungsergänzungsmittel, wie einem Nahrungsergänzungsmittel, das omega-3-Fettsäuren bereitstellt, verabreicht wird.

## Revendications

1. Chocolat ou produit de chocolat composé comprenant des ingrédients fonctionnels ajoutés comprenant la vitamine C, la vitamine E, le zinc, le cuivre et, facultativement, une xanthophylle, le chocolat ou produit de chocolat composé ayant une enveloppe et une garniture dans lesquelles au moins la vitamine C, la vitamine E et la xanthophylle sont comprises dans la garniture et dans lesquelles l'un ou les deux parmi le zinc et le cuivre sont présents dans l'enveloppe, et les ingrédients sont essentiellement chimiquement stables et ne produisent pas un effet sur la texture ou le goût détectable par un consommateur typique pendant la durée de conservation du chocolat ou produit de chocolat composé, à condition que la concentration de la vitamine C ajoutée ne dépasse pas environ 75 mg/g, celle de la vitamine E ajoutée environ 45 mg/g, celle du zinc ajouté environ 3 mg/g, celle du cuivre ajouté environ 0,4 mg/g et celle de la xanthophylle ajoutée environ 2,6 mg/g du chocolat ou produit de chocolat composé.

2. Chocolat ou produit de chocolat composé de la revendication 1, dans lequel la vitamine C est ajoutée sous la forme d'un ester de palmityle, lauryle, cocoyle, oléyle ou stéaryle.

3. Chocolat ou produit de chocolat composé de la revendication 1 ou 2, dans lequel la vitamine C est ajoutée sous la forme d'un ester de palmityle.

4. Chocolat ou produit de chocolat composé de l'une quelconque des revendications précédentes, dans lequel la xanthophylle facultativement ajoutée est la lutéine ou une combinaison de lutéine et de zéaxanthine.

5. Chocolat ou produit de chocolat composé de l'une quelconque des revendications précédentes, dans lequel la vitamine E, le cuivre et, si elle est incluse, la xanthophylle sont ajoutées surface d'ingrédients encapsulés.

6. Chocolat ou produit de chocolat composé de la revendication 5, dans lequel le zinc est également ajouté sous la forme d'un ingrédient encapsulé.

7. Chocolat ou produit de chocolat composé de la revendication 5 ou 6 dans lequel la taille de particule moyenne des ingrédients encapsulés est supérieure à environ 100 µm.

8. Chocolat ou produit de chocolat composé de la revendication 5 ou 6 dans lequel le cuivre et le zinc, ou la xanthophylle et la vitamine E sont ajoutés après co-encapsulation.

9. Chocolat ou produit de chocolat composé de l'une quelconque des revendications précédentes, dans lequel :
a) le zinc est ajouté sous forme encapsulée ou non encapsulée d'oxyde de zinc ou de sel de zinc sous la forme d'un sel de sulfate, chlorure, acétate, gluconate, ascorbate, citrate, aspartate, picolinate, orotate ou transferrine ;
b) le cuivre est ajouté sous la forme d'oxyde cuprique ou de sel cuprique comprenant le L-alaninate cuprique, le carbonate cuprique, le chlorure cuprique, le citrate cuprique, le gluconate cuprique, le glycinate cuprique, l'oxyde cuprique, le salicylate cuprique, le sulfate cuprique ou le tartrate cuprique encapsulé ; et
c) la vitamine E est ajoutée sous la forme d'acétate de DL-α-tocophéryle, DL-α-tocophérol, acétate de d-α-tocophéryle, d-α-tocophérol ou vitamine E isolés à partir de sources naturelles.

10. Chocolat ou produit de chocolat composé de l'une quelconque des revendications précédentes, dans lequel un constituant de sucre normal tel que le saccharose ou le lactose contenu dans l'enveloppe ou la garniture est supplémenté ou remplacé totalement ou en partie par l'un ou plusieurs d'un autre glucide tel qu'un ou plusieurs composés choisis dans le groupe constitué des fructose, tagatose, lactose, isomaltose, tréhalose, polydextrose et inuline ; un édulcorant de charge tel qu'un ou plusieurs composés choisis dans le groupe constitué des sorbitol, mannitol, xylitol, érythritol, maltitol, isomalt et lactitol ; et un édulcorant intense tel qu'un ou plusieurs composés choisis dans le groupe constitué des acésulfame potassium, alitame, aspartame, sel d'aspartate d'acésulfame, cyclamate, néotame, saccharine, stévia, sucralose et thaumatine.

11. Chocolat ou produit de chocolat composé unique ou pluralité de chocolats ou produits de chocolat composés selon l'une quelconque des revendications précédentes, destinés à une consommation quotidienne, comprenant :
a) 12 à 2000 mg de vitamine C ajoutée, 2,5 à 1000 mg de vitamine E ajoutée, 1,5 à 40 mg de zinc ajouté, 0,15 à 10 mg de cuivre ajouté et, facultativement, 1,5 à 33 mg de xanthophylle ajoutée ; ou
b) 180 à 1000 mg de vitamine C ajoutée, 30 à 500 mg de vitamine E ajoutée, 5 à 30 mg de zinc ajouté, 1 à 2 mg de cuivre ajouté et 7 à 20 mg de xanthophylle ajoutée ; ou
c) 500 mg de vitamine C ajoutée, 294 mg de vitamine E ajoutée, 25 mg de zinc ajouté, 1,6 mg de cuivre ajouté et 12 mg de xanthophylle ajoutée.

12. Procédé de production du chocolat ou produit de chocolat composé de l'une quelconque des revendications précédentes, comprenant la fabrication d'un chocolat ou produit de chocolat composé et l'étape supplémentaire d'ajout de quantités appropriées d'ingrédients fonctionnels de vitamine C, vitamine E, zinc, cuivre et, facultativement, xanthophylle à du chocolat liquide ou des masses de chocolat composé utilisées pour préparer l'enveloppe et la garniture, l'étape additionnelle étant conduite suite à toutes les étapes de broyage.

13. Chocolat ou produit de chocolat composé unique ou pluralité de chocolats ou produits de chocolat composés de l'une quelconque des revendications 1 à 11, pour utilisation dans la prévention et/ou le traitement diététique de maladies oculaires, ou pour favoriser la santé générale des yeux, le chocolat ou produit de chocolat composé unique ou la pluralité de chocolats ou produits de chocolat composés étant pour administration quotidienne à un sujet.

14. Produit(s) de la revendication 13, la maladie étant traitée ou prévenue est la dégénérescence maculaire liée à l'âge où la formation d'une cataracte.

15. Produit(s) de la revendication 13, le chocolat ou produit de chocolat composé unique ou la pluralité de chocolats ou produits de chocolat composés selon l'une quelconque des revendications 1 à 11 étant co-administrés avec un autre supplément nutritionnel, tel qu'un supplément nutritionnel qui fournit des acides gras oméga-3.
